**Europäisches Patentamt**

**European Patent Office**

**Office européen des brevets**

(11) Veröffentlichungsnummer: **0 211 095**
**B1**

(12) # EUROPÄISCHE PATENTSCHRIFT

(45) Veröffentlichungstag der Patentschrift:
28.06.89

(51) Int. Cl.⁴: **A61K 35/78**

(21) Anmeldenummer: **85109711.3**

(22) Anmeldetag: **02.08.85**

(54) **Arzneimittel enthaltend Extraktivstoffe aus Pflanzen oder Pflanzenteilen der Art Leptospermum scoparium.**

(43) Veröffentlichungstag der Anmeldung:
**25.02.87 Patentblatt 87/9**

(45) Bekanntmachung des Hinweises auf die Patenterteilung:
**28.06.89 Patentblatt 89/26**

(84) Benannte Vertragsstaaten:
**AT BE CH DE FR GB IT LI LU NL SE**

(56) Entgegenhaltungen:

(73) Patentinhaber: **Stirnadel, Alfred, Zweibrücker Strasse 63, D-6660 Zweibrücken 15(DE)**

(72) Erfinder: **Stirnadel, Alfred, Zweibrücker Strasse 63, D-6660 Zweibrücken 15(DE)**
Erfinder: **Stirnadel, Ute Helga, Zweibrücker Strasse 63, D-6660 Zweibrücken 15(DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90(DE)**

## Beschreibung

Extraktivstoffe aus pflanzlichem Material, wie Blättern, Blüten und Früchten, werden seit jeher zur Behandlung der verschiedensten Erkrankungen verwendet. Die in den Pflanzen enthaltenen Wirkstoffe können durch Extrahieren der Pflanzenteile mit Wasser, Alkohol oder anderen organischen Lösungsmitteln bzw. Mischungen davon, oder auch durch Wasserdampfdestillation gewonnen werden.

Es wurde nun überraschenderweise festgestellt, daß die Pflanzen der Art Leptospermum scoparium, die zur Familie der Myrtaceen gehört, in ihren Blüten, Knospen, Blättern und Stengeln Wirkstoffe hauptsächlich in Form etherischer Öle enthalten, die bereits in relativ geringer Menge oral appliziert zur Bekämpfung degenerativer Erscheinungen, wie Arthrose sowie zur Behandlung von Tumoren, Leukämie und Multipler Sklerose geeignet sind.

Gegenstand der Erfindung sind Arzneimittel, die Extraktivstoffe von Pflanzen oder Pflanzenteilen der Art Leptospermum scoparium enthalten.

Die Extraktiv- oder Wirkstoffe aktivieren offensichtlich die natürlichen Abwehrkräfte des Organismus gegen degenerative Vorgänge bzw. kanzerogene Erscheinungen im Körper. Diese neu erkannte Wirkung ist umso überraschender, als einerseits bekanntlich die Fachwelt seit vielen Jahrhunderten die verschiedensten Pflanzen und deren Teile auf das Vorhandensein von Stoffen mit therapeutischer Wirkung hin untersucht hat und andererseits die Pflanzen der Art Leptospermum scoparium seit langem bekannt sind. Leptospermum scoparium gedeiht in Australien und Neuseeland und wird auch in Europa wegen der schönen roten oder rosa Blüten als Zierpflanze geschätzt.

Vorzugsweise enthält das Arzneimittel Extraktivstoffe von Hybridsorten der Art Leptospermum scoparium. Die Hybridsorten werden durch Kreuzung oder Mutation erhalten. Bekannte und im Handel erhältliche Hybridsorten sind:

| | |
|---|---|
| BIG RED | JUBILEE |
| BLOSSOM | PINK PEARL |
| BURGUNDY QUEEN | SPECTRECOLOUR |
| FANTASIA | SUNRAYSIA |
| CHERRY RIPE | WINTER CHEER |
| CORAL CANDY | CRIMSON GLORY |

Zur Herstellung der Arzneimittel werden zerkleinerte Blätter, Stengel und/oder Blüten von Leptospermum scoparium mit Ethanol oder wäßrigem Ethanol extrahiert; nach dem Abtrennen der Pflanzenteile wird der Extrakt auf die gewünschte Konzentration verdünnt. Vorzugsweise wird das pflanzliche Material kurz vor der Vollblüte verwendet, wobei man die Pflanzenteile ohne die Wurzeln zerkleinert und mit Ethanol oder wäßrigem Ethanol, insbesondere mit 43%igem Ethanol in der Hitze, vorzugsweise durch Erhitzen unter Rückfluß, in an sich bekannter Weise extrahiert. Es können hierbei die frischen oder schonend getrockneten zerkleinerten Pflanzen bzw. deren Teile ohne die Wurzeln Verwendung finden.

Eine andere Art der Herstellung der Arzneimittel besteht darin, daß man die Extraktivstoffe mittels Wasserdampfdestillation des Ausgangsmaterials als etherisches Öl gewinnt.

Das gewonnene Decoctum, d. h. der alkoholische bzw. wäßrigalkoholische Extrakt kann in der gewünschten Verdünnung direkt appliziert werden. Wegen der hohen Aktivität der Extraktivstoffe aus Leptospermum scoparium eignet sich der Extrakt besonders zur Anwendung in homöopathischer Verdünnung entsprechend den Vorschriften der homöopathischen Arzneibücher, insbesondere entsprechend Vorschrift 19e, in einer D1 - D6-Verdünnung (Potenz), wobei dann im allgemeinen dreimal täglich 5 bzw. 15 bis 25 Tropfen vor dem Essen einzunehmen sind; dies entspricht 1 - 4 ml/Tag je 70 kg Körpergewicht.

Die Urtinktur kann als Phytopharmakon auch direkt oder 1 : 10 verdünnt in 30%igem Ethanol oder in 1%iger Kochsalzlösung zur oralen Applikation gelangen. In diesem Falle wären dreimal täglich 10 bis 25 Tropfen empfohlen.

Beispiel 1

200 g frisch gewonnene, klein geschnittene Blätter mit dünnen Stengeln einer Hybridsorte von Leptospermum scoparium werden mit 600 g 43%igem wäßrigem Ethanol versetzt. Der Ansatz wird zum Sieden erhitzt und 30 Minuten lang unter Rückfluß gehalten. Nach dem Abkühlen läßt man den Ansatz 24 Stunden unter Verschluß stehen, worauf durch Abpressen und Filtrieren die erhaltene Lösung der Extraktivstofe isoliert und gereinigt wird.

Zur Applikation kann der erhaltene Extrakt unverdünnt oder im Verhältnis 1 : 10 oder 1 : 100 mit 30%igem Ethanol verdünnt, verwendet werden. Zweckmäßigerweise werden 20 Tropfen des verdünnten Extraktes oral eingenommen. Dies sind 3 bis 4 ml je Tag bei 70 kg Körpergewicht. Wird die Herstellung einer Verdünnung nach den Lehren der Homöopathie gewünscht, so werden 3 Teile des filtrierten Extraktes mit 7 Teilen 30%igem Ethanol versetzt, worauf dann diese 1. Dezimalverdünnung (D1) im Verhältnis 1 : 9 nochmals mit 15 - 30%igem Ethanol verdünnt wird.

Die so erhaltenen D2-Verdünnung kann dann direkt oder nach weiterer Verdünnung zur Behandlung des Patienten oral appliziert werden.

Beispiel 2

100 g getrocknetes und gemahlenes Pflanzenmaterial (Stengel und Blätter) einer Hybridsorte von Leptospermum scoparium werden mit 1 l 43%igem Ethanol, wie in Beispiel 1 beschrieben, unter Rückfluß extrahiert. Nach Abkühlen und 24stündigem Stehenlassen wird abgepreßt und filtriert.

Da der erhaltene Extrakt bereits einer D1-Verdünnung entspricht, wird er zur Gewinnung einer D2-Verdünnung mit 30%igem Ethanol im Verhältnis 1 : 9 Teilen versetzt. Wenn gewünscht, wird 1 Teil der so erhaltenen Lösung nochmals mit 9 Teilen 15%igen Ethanols zur 3. Dezimalverdünnung versetzt. Die auf diese Weise erhaltenen Verdünnungen bzw. Potenzen D1 - D3 können dem Patienten oral appliziert werden.

Die so gewonnenen verdünnten Extrakte aus Leptospermum scoparium hybr. zeigen bereits nach einer regelmäßigen Applikation von 3 - 6 Wochen deutliche Erfolge. Das Mittel ist gut verträglich; es besitzt insbesondere in diesen Konzentrationen keine nachteiligen Wirkungen auf den Magen- und Darmtrakt.

Beispiel 3

Das aus den Pflanzenteilen der Leptospermum scoparium hybr.-Züchtungssorten durch Destillation gewonnene etherische Öl wird 1 : 10, 1 : 100, 1 : 000 und 1 : 10 000 mit Ethanol oder Oleum olivea verdünnt und kann so appliziert werden:
3 x 3 - 5 Tropfen bzw. 3 x 15 - 20 Tropfen.

**Patentansprüche**

1. Arzneimittel enthaltend Extraktivstoffe von Pflanzen oder Pflanzenteilen der Art Leptospermum scoparium.

2. Arzneimittel nach Anspruch 1, dadurch **gekennzeichnet,** daß es Extraktivstoffe von Hybridsorten der Art Leptospermum scoparium enthält.

3. Verfahren zur Herstellung des Arzneimittels nach Anspruch 1 oder 2, dadurch **gekennzeichnet,** daß man zerkleinerte Blätter, Stengel und/oder Blüten von Leptospermum scoparium oder von Hybridsorten davon mit Ethanol oder wäßrigem Ethanol extrahiert und nach dem Abtrennen der Pflanzenteile den Extrakt auf die gewünschte Konzentration verdünnt.

4. Verfahren nach Anspruch 3, dadurch **gekennzeichnet,** daß man die Pflanzenteile in der Hitze unter Rückfluß extrahiert.

5. Verfahren zur Herstellung des Arzneimittels nach Anspruch 1 oder 2, dadurch **gekennzeichnet,** daß man die Extraktivstoffe mittels Wasserdampfdestillation des Ausgangsmaterials als etherisches Öl gewinnt.

6. Verwendung der Extraktivstoffe von Pflanzen oder Pflanzenteilen der Art Leptospermum scoparium oder von Hybridsorten dieser Art zur Herstellung eines Arzneimittels für die Behandlung degenerativer Erscheinungen des Organismus wie Arthrose sowie für die Behandlung von Tumoren, Leukämie und Multipler Sklerose.

**Revendications**

1. Médicament contenant des extraits de plantes ou de parties de plantes du genre Leptospermum scoparium.

2. Médicament selon la revendication 1, caractérisé en ce qu'il contient des extraits de variétés hybrides du genre Leptospermum scoparium.

3. Procédé de préparation du médicament selon la revendication 1 ou 2, caractérisé en ce que les feuilles, les tiges et/ou les fleurs broyées, de Leptospermum scoparium ou de ses variétés hybrides sont extraites à l'éthanol aqueu et après séparation des parties des plantes, l'extrait est dilué à la concentration voulue.

4. Procédé selon la revendication 3, caractérisé en ce que les parties des plantes sont extraites à chaud sous reflux.

5. Procédé de préparation du médicament selon la revendication 1 ou 2, caractérisé en ce que les substances extraites sont obtenues sous forme d'essance par distillation à la vapeur d'eau de la matière première.

6. Utilisation des extraits de plantes ou de parties de plantes du genre Leptospermum scoparium ou de variétés hybrides de ce genre pour la préparation d'un médicament pour le traitement de processus dégénératifs de l'organisme tels que l'arthrose ainsi que pour le traitement de tumeurs, de la leucémie et de la sclérose en plaques.

**Claims**

1. A medicament containing extracts from plants or from plant parts of the species leptospermum scoparium.

2. The medicament of claim 1, characterized in that it contains extracts from hybrids of the species leptospermum scoparium.

3. A process for preparing the medicament of claim 1 or 2, characterized by extracting cut leaves, stems and/or blossoms of leptospermum scoparium or of hybrids thereof with ethanol or aqueous ethanol and, after separating the plant parts, diluting the extract to the desired concentration.

4. The process of claim 3, characterized by extracting the plant parts under heating to reflux.

5. A process for preparing a medicament according to claims 1 or 2, characterized in that the extracts are obtained by means of steam heat distillation of the starting material.

6. The use of extracts from plants or from plant parts of the species leptospermum scoparium or of hybrids of this species for the manufacture of a medicament for treatment of degenerative phenomena of the organism, such as arthritis, and for the treatment of tumors, leukemia and multiple sclerosis.